(19) Europäisches Patentamt
European Patent Office
Office européen des brevets

(11) Publication number : 0 509 768 A2

## (12) EUROPEAN PATENT APPLICATION

(21) Application number : 92303355.9

(22) Date of filing : 14.04.92

(51) Int. Cl.⁵ : C12N 15/29, C12N 15/54

(30) Priority : 17.04.91 US 687456

(43) Date of publication of application : 21.10.92 Bulletin 92/43

(84) Designated Contracting States : AT BE CH DE DK ES FR GB GR IT LI LU MC NL PT SE

(71) Applicant : ARIZONA BOARD OF REGENTS, ARIZONA STATE UNIVERSITY Tempe, Arizona 85287 (US)

(72) Inventor : Backhaus, Ralph A.
1530 West Juanita Circle
Mesa, Arizona 85202 (US)

(74) Representative : Coxon, Philip et al
Eric Potter & Clarkson St. Mary's Court St.
Mary's Gate
Nottingham NG1 1LE (GB)

(54) DNA clone of guayule rubber particle protein.

(57) Guayule rubber particles, which have high rubber transferase (RuT) activity, contain several characteristic proteins. The most abundant of these was purified by preparative gel electrophoresis. Designated the rubber particle protein (RPP), it is present in all guayule lines studied to date. A cDNA clone having a base sequence for the abundant guayule RPP has been developed and characterized and the amino acid sequence of the RPP has been determined.

FIG. 3

EP 0 509 768 A2

The present invention relates generally to bioengineering and more particularly to the isolation and elucidation of a rubber particle protein gene (RPP) the DNA template therefor, and the means and methods to replicate the DNA clone of the abundant guayule rubber particle protein in both friendly and foreign environments.

Rubber is found in more than two thousand plant species, but is limited to only a few families (See: Backhaus, Israel J. Bot, **34**, 283; and Archer et al (1963) Chemistry and Physics of Rubber-like Substances, Bateman, L. ed, pp 41-72, MacLaren, London). The rubber molecule is a polymer composed of 400-30,000 isoprene monomers enzymatically linked in a head-to-tail configuration. Guayule (Parthenium argentatum, Gray), a desert shrub, produces high molecular weight rubber molecules inside discrete rubber particles, which accumulate within the parenchyma cells of stembark tissues. Rubber transferase (RuT) [E.C. 2.5.1.20], the enzyme which causes the unique cis-1,4-polymerization of isoprene that leads to rubber formation in plants, is localized in these rubber particles (Cornish and Backhaus, Phytochem 29:3809-3813, (1990)). Only a few proteins are associated with guayule rubber particles and these have been recognized as being vital to rubber formation. One, presumably; is RuT.

The most abundant protein of rubber particles has been isolated, purified and characterized and is known as the guayule rubber particle protein (RPP).

Sodium dodecylsulphate polyacrylamide gel electrophoresis (SDS-PAGE) of proteins from rubber particles isolated from guayule stembark tissues, results in a simple but effective RPP purification procedure. The number of distinct protein bands associated with guayule rubber particles can vary from as few as one to several, depending on the age of the plant, time of harvest, genotype or isolation protocol used. However, in all cases studied, the single most abundant protein present is RPP having an apparent molecular weight of 48.5-52,000 Daltons. Examination of three different guayule lines, 593, 11591 and AZ 101, demonstrates the predominance of this protein (See: Cornish and Backhaus, op cit.).

RPP has been characterized with regard to its amino acid composition, pI, the chemistry of its oligosaccharide side chains and its relative position inside the rubber particle (Backhaus et al, Phytochem in press (1991)). Based on its apparent molecular weight, RPP contains about 444 amino acid residues. This may be an overestimate because the size of the glycosylated moiety of RPP has not yet been determined. The 150 electric point ("pI") predicted from the amino acid composition of RPP is 6:17. The observed pI for RPP, as determined by isoelectric focusing, is 6.2.

The low solubility of RPP in aqueous buffers and its strong association with rubber particles suggest it is a membrane protein. Protease digests of rubber particles following various detergent and heat treatments suggests that RPP occupies a protected position inside the rubber particle. The average hydropathy (GRAVY) for RPP, based on its amino acid composition, is -0.12 which lies within the range of GRAVY scores for a membrane protein. RPP also is known to be a glycoprotein (Backhaus et al, op cit, (1991)).

There is strong circumstantial evidence that RPP may be guayule RuT. Rubber particles can be isolated and purified from guayule stembark tissue in such a way that they maintain the capacity to carry out enzymatic rubber biosynthesis in vitro as described by Cornish and Backhaus, (op cit, (1990)). The enzyme responsible for this rubber biosynthetic activity is known as RuT and alternately as rubber polymerase and rubber cis, 1-4 polyprenyl transferase. The enzymatic aspects of RuT have been extensively described. (See: Backhaus, Israel J. Bot. 34: 283-293, (1985); Berndt, U.S. Government Res. Rep. AD-601, 729, (1963), Archer and Cockbain, Methods in Enzymology, 15:476-480, (1969) Archer and Audley, Advances in Enzymology, 29:221-257, (1967) and Lynen, Rubber Res. Inst. Malaya, 21(4):389-406, (1969).

RuT functional activity segregates with rubber particles which float when guayule stembark tissue homogenates are centrifuged above 2400 times gravity. Evidence for the purification of this enzyme from protein extracts of guayule rubber particles was described by Backhaus and Bess, in Randall, D.D. et al. (eds), Current Topics in Plant Biochemistry and Physiology, Vol 5:186, (1986); Cornish and Backhaus, op cit, (1990); Benedict et al., Plant Physiol. 92:816-821, (1990); and Backhaus et al., op cit, (1991). RuT activity has been associated with the abundant RPP, migrating at about 48.5-52,000 Daltons as determined by SDS-PAGE. RPP is essential for rubber biosynthesis in guayule, either because it is the rubber synthesizing enzyme or because it is a structural protein necessary for rubber formation.

The rubber particle protein (RPP) gene and the DNA template thereof are isolated and elucidated. The cDNA clone of the abundant guayule RPP is replicated.

The gene coding for the guayule RPP has been cloned. This protein is believed to be rubber transferase, the enzyme responsible for natural rubber biosynthesis. This protein was purified to homogeneity, cleaved with CNBr and the fragments sequenced from their N-termini. Oligonucleotides corresponding to known protein sequences were synthesized and used to prime plus and minus strand amplification of guayule bark cDNA, via the polymerase chain reaction (PCR). This resulted in a 92 bp fragment of DNA which, when sequenced, matched perfectly with the known protein sequence of RPP. Subsequently, this probe was used to isolate a partial cDNA clone from a guayule stembark, lambda ZAP cDNA library. Upon isolation, the full-length gene for RPP will be cloned into an expression vector and assayed for rubber transferase enzyme activity.

Initially, guayule stembark lambda ZAP cDNA libraries were screened with specific radioactively labeled oligonucleotide sequences ("probes") corresponding to known amino acid sequences of CNBr generated fragments of RPP. Positive clones were further screened with radioactive probes. Twenty eight clones were obtained which contained RPP gene sequences of various lengths. The longest clone, c17, was 823 base pairs (bp) long while the other twenty seven varied from 800 to 400 bp in length. Partial DNA sequencing showed the clones to all have sequences identical to the oligonucleotide probes and to the longer 823 kb clone. Nucleotide sequence analysis showed that c17 consisted of a 823 bp cDNA containing the 3′ region of RPP. The c17 insert contained an open reading frame of 507 bp which contained DNA sequences coding for CNBr fragments #2, #1 and #4, respectively, from the 5′ to 3′ direction. The 507 bp open reading frame encodes a 169 amino acid sequence equivalent to 18,958 Daltons which includes 2 methionines and 2 cysteines. There are 2 potential N-linked glycosylation sites in this region of RPP with the sequence Asn-X-Ser/Thr, wherein X can be any of the common 20 amino acids. In addition, c17 contains a stop codon (TAA) and a 3′-noncoding region of 313 bp.

The original source of the protein material for developing the cDNA clone c17 was stembark tissue from the 11591 line of guayule shrubs. Such tissue is rich in natural rubber and is readily available from guayule field research plots which are growing in and around the Phoenix metropolitan area. The lambda ZAP cDNA cloning system used herein is a well-known and commonly available lambda phage expression vector. Its construction arid restriction endonuclease map is described by Short, J.M. et al. Nucl. Acids Res. 16:7583-7600, (1988), Huse, W.D. and Hansen, C., Strategies 1:1-3, Sorge, J., Strategies 1:3-7 (1988), and associated techniques in Gubler, U. and Hoffman, B.J., Gene 25:263 (1983), Young, R.A. and Davis, R.W., Proc. Natl. Acad. Sci. USA 80:1194-1198 (1983) and Watson, C.J. and Jackson, J.F. DNA cloning; A practical approach 79-88, (1985).

The cloning of the cDNA for RPP and development of its protein sequence and structural domains as disclosed herein will permit structure-functional analysis and provide a foundation for study of its biosynthetic regulation and its role in rubber biosynthesis in guayule and other organisms. The expression of RPP,in natural or foreign host organisms can be regulated by placing RPP cDNA downstream of an appropriate 5′ promoter sequence and transferring the resulting recombinant RPP gene into the genome of that host. The effect of RPP gene expression on rubber biosynthesis can then be assessed. This invention is thus important to the agricultural, biological and chemical sciences and offers a basis for natural rubber production, which could be imparted to a wide variety of prokaryotic or eukaryotic systems by recombinant DNA methods. A DNA sequence encoding RPP or a protein having substantially the same biological activity as RPP, is also disclosed. The DNA sequences may be obtained from cDNA or genomic DNA, or may be prepared by DNA synthesis techniques.

Accordingly, a primary object of the present invention is to provide DNA sequences and recombinant DNA molecules coding for RPP.

Another object of the present invention is to provide new and improved processes for the production of polypeptides from DNA sequences and recombinant expression systems for such sequences.

A further object of the present invention is to provide vectors containing the proper DNA sequences and the cultures for producing the recombinant protein.

These and still further objects as will be readily discerned from the following detailed description of an exemplary embodiment thereof especially when read in conjunction with the accompanying drawing in which like parts bear like numerals throughout the several views.

In the drawings:

FIG. 1 depicts the amino acid sequences of fragments obtained from a cyanogen bromide digestion of the abundant guayule RPP;

FIG. 2 shows the chemically synthesized oligonucleotide DNA probes and/or primers in accordance with the present invention;

FIG. 3 displays the nucleotide sequence of the cDNA insert of the c17 clone of RPP.

In order that the invention herein described may be fully understood, the following definitions are provided.

**Nucleotide** means a monomeric unit of DNA or RNA consisting of a sugar moiety (Pentose), a phosphate, and a nitrogenous heterocyclic base. The base is linked to the sugar moiety via the glycosidic carbon (1′ carbon of the pentose) and that combination of base and sugar is called a "nucleoside". The base characterizes the nucleotide. The four DNA bases are adenine ("A"), guanine ("G"), cytosine ("C"), and thymine ("T"). The four RNA bases are A, G, C, and uracil ("U"). As is conventional for convenience in the structural representation of a DNA nucleotide sequence only one strand is shown in which A on one strand connotes T on its complement and G connotes C. Amino acids are shown either by a three letter or one letter abbreviation as follows:

| Abbreviated Designations | — Amino Acid |
|---|---|
| A Ala | Alanine |
| C Cys | Cysteine |
| D Asp | Aspartic acid |
| E Glu | Glutamic acid |
| F Phe | Phenylalanine |
| G Gly | Glycine |
| H His | Histidine |
| I Ile | Isoleucine |
| K Lys | Lysine |
| L Leu | Leucine |
| M Met | Methionine |
| N Asn | Asparagine |
| P Pro | Proline |
| Q Gln | Glutamine |
| R Arg | Arginine |
| S Ser | Serine |
| T Thr | Threonine |
| V Val | Valine |
| W Trp | Tryptophan |
| Y Tyr | Tyrosine |

**DNA Sequence** means a linear array of nucleotides connected one to the other by phosphodiester bonds between the 3′ and 5′ carbons of adjacent pentoses.

**Codon** means a DNA sequence of three nucleotides (a triplet) which encodes through mRNA an amino acid, a translation start signal or a translation termination signal. For example, the nucleotide triplets TTA, TTG, CTT, CTC, CTA and CTG encode for the amino acid leucine ("Leu"), TAG, TAA and TGA are translation stop signals and ATC is a translation start signal.

**Reading Frame** means the grouping of codons during the translation of mRNA into amino acid sequences. During translation the proper reading frame must be maintained. For example, the DNA sequence GCTGGTTGTAAG may be expressed in three reading frames or phases, each of which affords a different amino acid sequence:

GCT GGT TCT AAG-Ala-Gly-Cys-Lys

G CTG GTT GTA AG-Leu-Val-Val

GC TGG TTG TAA G-Trp-Leu-(STOP)

**Polypeptide** means a linear array of amino acids connected one to the other by peptide bonds between the alpha-amino and carboxyl groups of adjacent amino acids.

**Genome** means the entire DNA of a cell or a virus. It includes, inter alia, the structural gene coding for the polypeptides of the substance, as well as operator, promoter, terminator, enhancer and ribosome binding and interaction sequences.

**Gene** means a DNA sequence which encodes through its template or messenger RNA ("mRNA") a sequence of amino acids characteristic of a specific polypeptide.

**cDNA** means a complementary or copy DNA prepared by using mRNA as a template for synthesizing the first strand of DNA using reverse transcriptase, an appropriate oligonucleotide primer and a mixture of nucleotides.

**PCR** means a polymerase chain reaction whereby a specific DNA sequence, either genomic or cDNA, can be preferentially amplified by the enzyme Taq polymerase using synthetic, oligonucleotide sense and antisense primers, (which specify a target sequence), a mixture of nucleotides and a temperature thermocycling régime which allows sequential denaturing, annealing and synthesis of the target DNA between the primers.

**Transcription** means the process of producing mRNA from a gene or DNA sequence.

**Translation** means the process of producing a polypeptide from mRNA.

**Expression** means the process undergone by a gene or DNA sequence to produce a polypeptide and comprises a combination of transcription and translation.

**Plasmid** means a nonchromosomal double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for ampicillin resistance (AMP$^R$) transforms a cell previously sensitive to ampicillin into one which is resistant to it. A cell transformed by a plasmid is called a "transformant".

**Phage** or **Bacteriophage** means a bacterial virus, many of which consist of DNA sequences encapsidated in a protein envelope or coat ("capsid").

**Cloning Vehicle** means a plasmid, phage DNA, cosmid or other DNA sequence which is able to replicate in a host cell, characterized by one or a small number of endonuclease recognition sites at which such DNA sequences may be cut in a determinable fashion without attendant loss of an essential biological function of the DNA, e.g., replication, production of coat proteins or loss of promoter or binding sites, and which contain a marker suitable for use in the identification of transformed cells, e.g., ampicillin resistance. A cloning vehicle is often called a vector.

**Cloning** means the process of obtaining a population of organisms or DNA sequences derived from one such organism or sequence by asexual reproduction.

**Recombinant DNA Molecule** or **Hybrid DNA** means a molecule consisting of segments of DNA from different genomes which have been joined end-to-end outside of living cells and able to be maintained in living cells.

**Expression Control Sequence** means a sequence of nucleotides that controls and regulates expression of genes when operatively linked to those genes. They include the lac system, the beta-lactamase system, the trp system, the tac and trc systems, the major operator and promoter regions of phage lambda, the NOS promoter and terminator system, the 35S promoter and other sequences known to control the expression of genes of prokaryotic or eukaryotic cells and their viruses or combinations thereof.

**Guayule rubber particle - protein** (RPP), is a polypeptide localized in the rubber particles of guayule which is essential for rubber formation in that plant.

The present invention relates to DNA sequences and recombinant DNA molecules coding for the guayule RPP and processes for the production of those polypeptides, to recombinant expression systems for these sequences, to vectors containing them, to cultures producing the recombinant protein, and to the materials significant in its production. A DNA sequence encoding guayule RPP or a protein having substantially the same biological activity as guayule RPP is shown.

Although a variety of selection and DNA cloning techniques might potentially have been employed in our isolating and cloning of a DNA sequence of this invention, a selection strategy based on purified RPP was adopted.

**Purification and CNBr cleavage of RPP.**

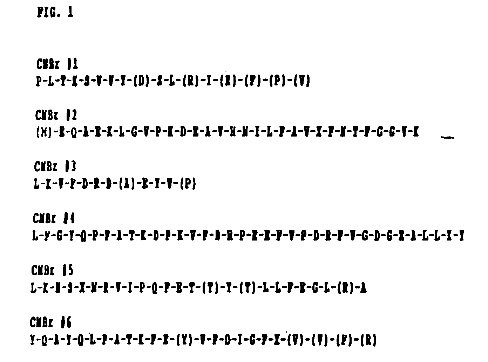

The source of RPP is from purified rubber particles which are isolated from guayule line 11591 stembark tissues by the procedure described by Cornish and Backhaus, op. cit,(1990) and Backhaus et al, op cit, (1991). Purified rubber particles were subjected to preparative SDS-PAGE to facilitate the purification of RPP, which could be observed in the gel as a protein band migrating with an apparent molecular weight of 48.5 - 52,000 Daltons. RPP was purified from gel bands by electroelution using a Model 422 Electroeluter (Bio-Rad) according to manufacturer's instructions. Purified RPP equivalent to at least 1000 pmoles was taken to dryness by lyophilization and dissolved in 150 μl 70% formic acid in an Eppendorf tube. To this 100μl of 70 μg/mL CNBr in 70% formic acid was added. The mixture was incubated in the dark at room temperature for 24 hours. The CNBr digested RPP was subjected to SDS-PAGE using a 16% acrylamide gel in a Tris-Tricine, 3-layer system as described by Schagger and Von Jagow, Anal. Biochem 166:368-379, (1987). Following electrophoresis, protein fragments were blotted onto PVDF (Millipore) membranes and visualized according to the method of Ploug et al. Anal. Biochem. 181:33-39, (1989). Areas of PVDF membranes containing stained peptides were excised and submitted for N-terminal amino acid sequencing (Univ. Calif. Davis, Protein Structure Lab). The results of amino acid sequencing of six distinct peptides fragments of RPP is shown in FIG. 1.

**Isolation of guayule stembark mRNA.**

Guayule stembark (10 g) was cut into 1 cm pieces and homogenized in 2 vol of quanidine buffer (8 M guanidine HCL, 20 mM MES pH 7, 20 mM EDTA, 50 mM mercaptoethanol) in a polytron for 2 minutes. The homogenate was extracted with 1 vol of phenol:chloroform and centrifuged for 45 minutes at 10,000 rpm (Sorval SS-34 rotor) at 15 C. The aqueous phase was transferred to a fresh tube and centrifuged for 10 min at 10,000 rpm at 15 C and the supernatant transferred to fresh tube. To this 0.7 vol of pre-cooled 100% ethanol and 0.2 vol of 1 M acetic acid was added. The sample was placed in -20 C overnight. The RNA was recovered by centrifugation for 10 min at 5,000 rpm at 4C. The pellet was washed with sterile 3M sodium acetate (pH 5.2) at room temperature and centrifuged for 5 min at 10,000 rpm. The pellet was washed with 70% ethanol, vacuum dried and dissolved in sterile water. Poly-A+ mRNA was purified from RNA by fractionation on oligo-dT cellulose according to methods described in Sambrook et al. Molecular Cloning, A laboratory Manual, 2nd Ed. 7.26-7.29 (1989).

**Construction of guayule stembark cDNA libraries.**

Guayule stembark mRNA was used to generate cDNA for construction of libraries in lambda ZAP, available in kit form from Strategene Inc., according to the protocol outlined by the manufacturer. Lambda ZAP is described by Short et al, Nucl., Acids Res. 16:7583-7600 (1988).

**PCR amplification of guayule RPP cDNA**

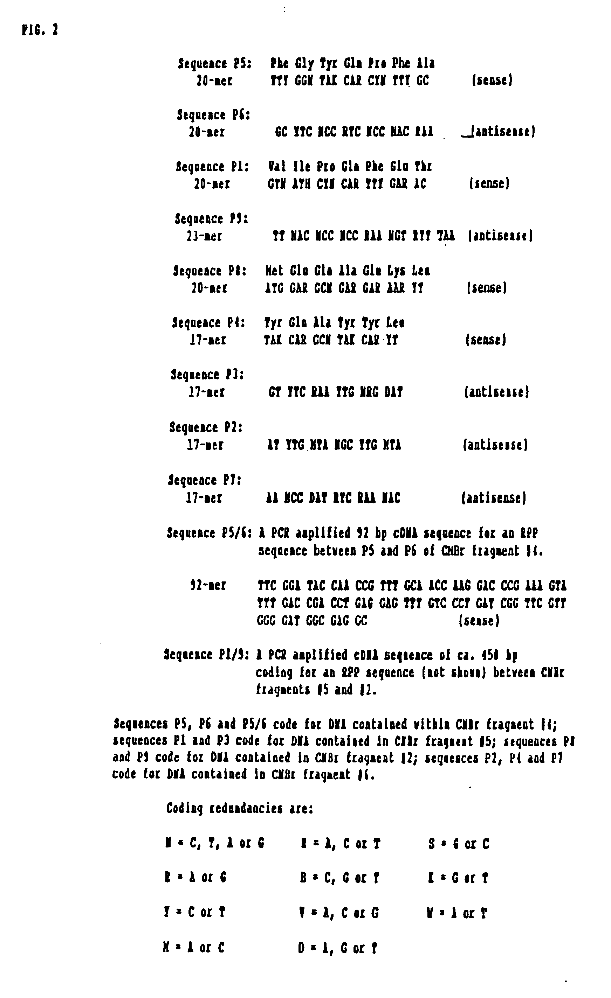

The guayule stembark cDNA libraries were initially screened with a 92 bp probe, "sequence P5/6" (FIG. 2) generated by PCR amplification of guayule cDNA. The first strand cDNA synthesis was performed by using the Strategene, cDNA synthesis kit and following the manufacturer's instructions. About 1.5 $\mu$g of guayule stembark mRNA was incubated at 37 C for 1 h in the presence of oligo-dT, reverse transcriptase and a mixture of nucleotides. The single stranded cDNA was precipitated by adding ammonium acetate (pH 4.5) to a final concentration of 0.5 M followed by 2 vols of ethanol. The mixture was centrifuged in a microcentrifuge at 14,000 rpm at room temperature for 10 min and the pellet was washed with 200 $\mu$l of 70% ethanol, dried under vacuum and redissolved in 50 $\mu$l of sterile water.

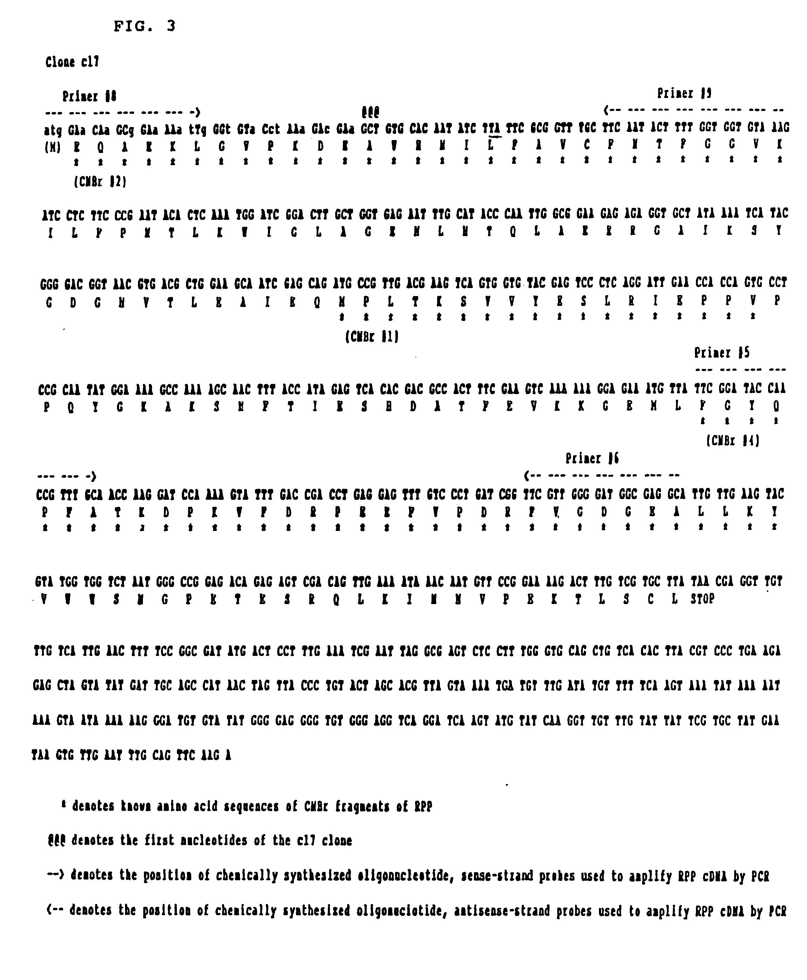

Sequence P5 (FIG. 2), a degenerate, 20-mer oligonucleotide primer corresponding to a sense strand DNA of CNBr fragment #4 and sequence P6 (FIG. 2), a degenerate, 20-mer oligonucleotide primer corresponding to an antisense strand DNA of CNBr fragment #4 (FIG. 1) were used to generate the 92 bp P5/6 sequence (FIG. 2). A 20 $\mu$l PCR reaction mixture containing 100 pmol of each primer, 1 $\mu$l of the cDNA mixture described above, 0.2 mM of each nucleotide and 0.5 $\mu$l REPLINASE (DuPont) in 50 mM Tris-HCl buffer (pH 9.0) containing 20 mM ammonium sulfate and 1.5 mM magnesium chloride was amplified under a thermalcycling régime of 94°C for 1 min, 55°C for 2 min and 72°C for 3 min for 39 cycles followed by 10 min at 72°C. The resulting mixture was run on an agarose gel to reveal a 92 bp fragment. DNA sequence analysis of the fragment revealed an exact match to the amino acid sequence of CNBr fragment #4 as shown in FIGS. 2 and 3.

Subsequent to this, radioactively labeled $^{32}$P probes of P5/6 were regenerated by the PCR reaction, using 50 pmol of each primer P5 and P6 and utilizing the purified P5/6 probe as the template DNA. This resulted in high specific activity probe which was used to screen the lambda ZAP cDNA library, according to the methods outlined in Sambrook, el al. (op cit).

Subsequent to determining the sequence of the c17 clone it was discovered that P5/6 encodes a 3' terminal region of the RPP gene (FIG. 3). Thus, when screening the cDNA library with P5/6 it resulted in the preferential isolation of only partial cDNA clones, such as c17. To isolate full-length cDNA clones a new PCR amplified probe, P1/9, has been developed. It is ca. 450 bp long and codes for an internal RPP sequence between primer sequence P1 (FIG. 2), a degenerate, 20-mer oligonucleotide sense strand coding for DNA contained in CNBr fragment #5 and primer sequence P9 (FIG. 2 and FIG.3), a degenerate 23-mer oligonucleotide antisense strand coding for DNA contained in fragment #2 and in the 5' region of c17. P1/9 will be used to screen cDNA libraries and should result in the identification of full-length cDNA clones. Since the same preparation of stembark cDNA was used as a template to obtain P1/9 it is anticipated that a cDNA clone containing P1/9 should also exist in the library. Moreover, since the P1/9, clone is 450 bp long, this would make it extend to the putative 5' end of the RPP, based on the expected length of the RPP gene which is anticipated to have an open reading frame of ca. 1200-1400 bp.

If necessary, alternative strategy will be to use the RACE method (Frohman et al. Proc. Natl. Acad Sci. USA 85:8998-9002, 1988) to clone the remaining 5' region of RPP. This will be possible because a large portion of the exact DNA sequence of the RPP gene is now known.

**Screening of cDNA libraries**

Plaques containing recombinant lambda ZAP cDNA's were transferred to membrane filters (Colony/Plaque Screen, DuPont) and subjected to an initial screening by hybridization to radioactively labeled P5/6 in 6X SSPC, 5X Denhardt's solution, 50% formamide, 0.5% SDS, 200 $\mu$g/ml salmon sperm DNA, and 10% dextran sulfate for 18 h at 42°C. Washes were for 2 times at 10 min in 2X SSC followed by 2 times at 20 min in 2X SSC plus 0.1% SDS. Positive plaques from the initial screen were rescreened on nitrocellulose (Schleicher and Schuell) by hybridization to radioactively labeled P5/6 in 6X SSPE, 5X Denhardt's solution, 0.5% SDS and 100 µg/ml salmon sperm DNA for 18 h at 42°C and washed the same as for the initial screens.

Clones which yielded positive responses to the second screen were then subjected to excision according to the lambda ZAP protocol. Plasmid DNA was isolated by standard miniprep procedures and separated on agarose gels. Those colonies resulting in Bluescript plasmids containing the largest cDNA inserts were further characterized by DNA sequencing.

**DNA sequencing**

DNA sequencing employed the dideoxy chain termination technique, utilizing SEQUENASE (U.S. Biochemical Corp) according to manufacturer's instructions.

The cleavage of RPP by CNBr resulted in at least six distinct peptide fragments which could be sequenced from their N-terminus. (FIG. 1). Amino acid sequences ranged from 12 to 36 residues in length. Of those, peptide fragments #2, #4, #5 and #6 resulted in deduced DNA stretches of at least 17 bp with low degeneracy (FIG. 2). Oligonucleotides corresponding to those portions were synthesized and used to generate larger guayule cDNA fragments by PCR amplification. Of those, two successful PCR products were made. A 92 bp sequence, P5/6 which matched with the known CNBr fragment #4 (FIG. 2) and a ca. 450 bp sequence, P1/9 which contained 5′ regions of RPP sequence, bridging CNBr fragments #2 and #5. Sequence P5/6 was used as a probe to successfully isolate a large cDNA clone c17 which contained an 823 bp insert coding for the 3′ end of the RPP gene (FIG. 3). Starting with the nucleotides GCT GTG CAC AAT, coding for AVHN, where A is 1, V is 2, etc. the c17 clone contains an open reading frame of 507 bp which encodes a polypeptide of 169 amino acids with a molecular weight equivalent to 18,958 Daltons. This polypeptide shows exact homology with three of the six CNBr fragments, #2, #1 and #4, going from the 5′ to 3′ direction. As expected, each of these fragments is preceded by a methionine which is the cleavage site for CNBr digestion. However, the region upstream from AVHN is deduced from the known sequence of CNBr fragment #2 and did not appear in c17. Subsequent cloning of larger cDNA inserts will verify this exact sequence. In addition, c17 contains 2 cysteines, at position 10 and at the extreme C-terminus, position 168. The c17 polypeptide also contains two possible N-linked glycosylation sites at positions 53 and 89 from the AVHN site. This is consistent with the structure of guayule RPP which is known to be a glycoprotein. The c17 RPP region is negatively charged with a deduced pKa of ca. 2.2. The remainder of the c17 clone contains a TAA stop codon and a 3′ noncoding sequence of 313 bp which is AT-rich as expected for a termination sequence.

A computer search of GENBANK, EMBL and NBRF did not result in homology with any existing DNA or amino acid sequences corresponding to the 169 amino acid sequence in the open reading frame of c17. These sequences do not match any of the known sequences for Hevea proteins putatively involved with rubber biochemistry, including REF and FPP synthetase (Dennis and Light, J. Biol. Chem. 264: 18608; Light and Dennis, J. Biol. Chem. 264: 18589, (1989)). The exact role of guayule RPP remains to be determined.

As shown above, the present invention is predicated upon a selection strategy based upon guayule RPP in which guayule RPP was purified and the amino acid sequence of various fragments of that protein was determined. Based on those protein sequences, several oligonucleotide DNA probes were synthesized corresponding to those regions of RPP which had minimal nucleotide degeneracy. These probes were then used to screen a guayule bark cDNA library comprising E. coli cells containing guayule cDNA sequences inserted into a phage cloning vector.

The cDNA sequences of this invention can be operatively linked to expression control sequences and used in various eukaryotic or prokaryotic host cells to produce polypeptides coded for by them. The cDNA sequences of this invention, are also useful as probes to screen genomic DNA sequences coding for polypeptides. These genomic sequences, like the above cDNA sequences of this invention, are then useful to produce the polypeptides coded for by them.

From the foregoing, it is readily apparent that a useful embodiment of the present invention has been herein described and illustrated which fulfills all of the aforestated objectives in a remarkably unexpected fashion. It is of course understood that such modifications, alterations and adaptations as may readily occur to the artisan confronted with this disclosure are intended within the spirit of this disclosure which is limited only by the scope of the claims appended hereto.

**Claims**

1.    Guayule RPP clone c17 containing an 823 basepair insert as shown in FIG. 3.

2.  The cDNA sequence having the nucleotide sequence shown in FIG. 3 which encodes guayule RPP having the amino acid sequence shown in FIG. 3.

3.  A cDNA sequence consisting of a nucleotide sequence encoding guayule RPP which has the amino acid sequence of FIG. 3.

4.  DNA sequences which hybridize to the cDNA sequence of guayule RPP.

5.  An isolated DNA sequence which encodes for a guayule RPP wherein the RPP contains an amino acid sequence comprising: Leu - Lys - Val - Phe - Asp - Arg - Asp - Ala - Glu - Tyr - Val -.

6.  An isolated DNA sequence which encodes for a guayule RPP wherein the RPP contains an amino acid sequence comprising: Leu - Lys - Asn - Ser - X - Asn - Arg - Val - Ile - Pro - Gln - Phe - Glu - Thr - Thr - Tyr - Thr - Leu - Leu - Phe - Glu - Gly - Leu -.

7.  An isolated DNA sequence which encodes for a guayule RPP wherein the RPP contains an amino acid sequence comprising: Tyr - Gln - Ala - Tyr - Gln - Leu - Phe - Ala - Thr - Lys - Phe - Glu - Try - Val - Phe - Asp - Ile - Gly - Phe -.

8.  The recombinant DNA molecule according to claim 2 in which said DNA sequence is operatively linked to an expression control sequence in the molecule.

9.  The recombinant DNA molecule according to claim 5 in which said DNA sequence is operatively linked to an expression control sequence in the molecule.

10. The recombinant DNA molecule according to claim 6 in which said DNA sequence is operatively linked to an expression control sequence in the molecule.

11. The recombinant DNA molecule according to claim 7 in which said DNA sequence is operatively linked to an expression control sequence in the molecule.

12. The recombinant DNA molecule according to claim 8 wherein said expression control sequence is selected from the group consisting of the lac system, the β-lactamase system, a trp system, a trc system, major operator and promoter regions of phage lambda, the NOS promoter and terminator system, the 35S promoter, and other sequences which control the expression of genes of prokaryotic or eukaryotic cells and their viruses.

13. The recombinant DNA molecule according to claim 9 wherein said expression control sequence is selected from the group consisting of the lac system, the β-lactamase system, a trp system, a trc system, major operator and promoter regions of phage lambda, the NOS promoter and terminator system, the 35S promoter, and other sequences which control the expression of genes of prokaryotic or eukaryotic cells and their viruses.

14. The recombinant DNA molecule according to claim 10 wherein said expression control sequence is selected from the group consisting of the lac system, the β-lactamase system, a trp system, a trc system, major operator and promoter regions of phage lambda, the NOS promoter and terminator system, the 35S promoter, and other sequences which control the expression of-genes of prokaryotic or eukaryotic cells and their viruses.

15. The recombinant DNA molecule according to claim 11 wherein said expression control sequence is selected from the group consisting of the lac system, the β-lactamase system, a trp system, a trc system, major operator and promoter regions of phage lambda, the NOS promoter and terminator system, the 35S promoter, and other sequences which control the expression of genes of prokaryotic or eukaryotic cells and their viruses.

FIG. 1

CNBr #1
P-L-T-X-S-V-V-Y-(D)-S-L-(R)-I-(E)-(F)-(P)-(V)

CNBr #2
(M)-E-Q-A-R-K-L-G-V-P-K-D-E-A-V-H-N-I-L-P-A-V-X-P-N-T-P-G-G-V-K    _

CNBr #3
L-X-V-P-D-R-D-(A)-E-Y-V-(P)

CNBr #4
L-P-G-Y-Q-P-P-A-T-K-D-P-K-V-P-D-R-P-R-R-P-V-P-D-R-P-V-G-D-G-R-A-L-L-I-Y

CNBr #5
L-X-N-S-X-M-R-V-I-P-Q-P-E-T-(T)-Y-(T)-L-L-P-E-G-L-(R)-A

CNBr #6
Y-Q-A-Y-Q-L-P-A-T-K-P-R-(Y)-V-P-D-I-G-P-X-(V)-(V)-(P)-(R)

FIG. 2

Sequence P5:  Phe Gly Tyr Gln Pro Phe Ala
20-mer    TTY GGN TAX CAR CYN TTY GC   (sense)

Sequence P6:
20-mer    GC YTC NCC RTC NCC NAC RAA  (antisense)

Sequence P1:  Val Ile Pro Gln Phe Glu Thr
20-mer    GTN ATH CYN CAR TTY GAR AC  (sense)

Sequence P9:
23-mer    TT NAC NCC NCC RAA NGT RTY TAA (antisense)

Sequence P8:  Met Glu Gln Ala Glu Lys Leu
20-mer    ATG GAR GCN GAR GAR AAR YT  (sense)

Sequence P4:  Tyr Gln Ala Tyr Tyr Leu
17-mer    TAX CAR GCN TAX CAR YT  (sense)

Sequence P3:
17-mer    GT YTC RAA YTG NRG DAT  (antisense)

Sequence P2:
17-mer    AT YTG NTA NGC YTG NTA  (antisense)

Sequence P7:
17-mer    AA NCC DAT RTC RAA NAC  (antisense)

Sequence P5/6: A PCR amplified 92 bp cDNA sequence for an RPP
sequence between P5 and P6 of CNBr fragment #4.

92-mer    TTC GGA TAC CAA CCG TTT GCA ACC AAG GAC CCG AAA GTA
TTT GAC CGA CCT GAG GAG TTT GTC CCT GAT CGG TTC GTT
GGG GAT GGC GAG GC    (sense)

Sequence P1/9: A PCR amplified cDNA sequence of ca. 450 bp
coding for an RPP sequence (not shown) between CNBr
fragments #5 and #2.

Sequences P5, P6 and P5/6 code for DNA contained within CNBr fragment #4;
sequences P1 and P9 code for DNA contained in CNBr fragment #5; sequences P8
and P9 code for DNA contained in CNBr fragment #2; sequences P2, P4 and P7
code for DNA contained in CNBr fragment #6.

Coding redundancies are:

N = C, T, A or G     H = A, C or T     S = G or C

R = A or G          B = C, G or T     K = G or T

Y = C or T          V = A, C or G     W = A or T

M = A or C         D = A, G or T

FIG. 3

Clone cl7

```
Primer #8                                                          Primer #9
--- --- --- --- --- --- -)                   ###          (-- --- --- --- --- --- --- --
atg GAa CAa GCg GAa AAa tTg GGt GTa Cct AAa GAc GAa GCT GTG CAC AAT ATC TTA TTC ECG GTT TGC TTC AAT ACT TTT GGT GGT GTA AAG
(M)  E   Q   A   E   K   L   G   V   P   K   D   E   A   V   R   N   I   L   F   A   V   C   F   N   T   F   G   G   V   K
     *   *   *   *   *   *   *   *   *   *   *   *   *   *   *   *   *   *   *   *   *   *   *   *   *   *   *   *   *   *
     (CNBr #2)

ATC CTC TTC CCG AAT ACA CTC AAA TGG ATC GGA CTT GCT GGT GAG AAT TTG CAT ACC CAA TTG GCG GAA GAG AGA GGT GCT ATA AAA TCA TAC
I   L   F   P   N   T   L   K   W   I   G   L   A   G   E   N   L   N   T   Q   L   A   E   R   R   G   A   I   K   S   Y

GGG GAC GGT AAC GTG ACG CTG GAA GCA ATC GAG CAG ATG CCG TTG ACG AAG TCA GTG GTG TAC GAG TCC CTC ACG ATT GAA CCA CCA GTC CCT
G   D   G   N   V   T   L   E   A   I   E   Q   M   P   L   T   K   S   Y   V   I   E   S   L   R   I   E   P   P   V   P
                                                *   *   *   *   *   *   *   *   *   *   *   *   *   *   *   *   *   *   *
                                                (CNBr #1)

                                                                                    Primer #5
                                                                                    --- --- --- ---
CCG CAA TAT GGA AAA GCC AAA AGC AAC TTT ACC ATA GAG TCA CAC GAC GCC ACT TTC GAA GTC AAA AAA GGA GAA ATG TTA TTC GGA TAC CAA
P   Q   Y   G   K   A   K   S   N   F   T   I   E   S   H   D   A   T   F   E   V   K   K   G   E   N   L   F   G   Y   Q
                                                                                                        *   *   *   *
                                                                                                        (CNBr #4)

                                                                Primer #6
--- --- -)                                              (-- --- --- --- --- --- --
CCG TTT GCA ACC AAG GAT CCA AAA GTA TTT GAC CGA CCT GAG GAG TTT GTC CCT GAT CGG TTC GTT GGG GAT GGC GAG GCA TTG TTG AAG TAC
P   F   A   T   K   D   P   K   V   F   D   R   P   E   E   F   V   P   D   R   F   V   G   D   G   E   A   L   L   K   Y
*   *   *   *   *   *   *   *   *   *   *   *   *   *   *   *   *   *   *   *   *   *   *   *   *   *   *   *   *   *   *

GTA TGG TGG TCT AAT GGG CCG GAG ACA GAG ACT CGA CAG TTG AAA ATA AAC AAT GTT CCG GAA AAG ACT TTG TCG TGC TTA TAA CGA GGT TGT
V   W   W   S   N   G   P   E   T   E   S   R   Q   L   K   I   N   N   V   P   E   K   T   L   S   C   L   STOP
```

TTG TCA TTG AAC TTT TCC GGC GAT ATG ACT CCT TTG AAA TCG AAT TAG GCG AGT CTC CTT TGG GTG CAC CTG TCA CAC TTA CGT CCC TGA ACA

GAG CTA GTA TAT GAT TGC AGC CAT AAC TAG TTA CCC TGT ACT AGC ACG TTA GTA AAA TGA TGT TTG ATA TGT TTT TCA AGT AAA TAT AAA AAT

AAA GTA ATA AAA AAG GGA TGT GTA TAT GGG GAC GGG TGT GGG AGG TCA GGA TCA AGT ATG TAT CAA GGT TGT TTG TAT TAT TCG TGC TAT GAA

TAA GTG TTG AAT TTG CAG TTC AAG A

* denotes known amino acid sequences of CNBr fragments of RPP

### denotes the first nucleotides of the cl7 clone

—) denotes the position of chemically synthesized oligonucleotide, sense-strand probes used to amplify RPP cDNA by PCR

(-- denotes the position of chemically synthesized oligonucleotide, antisense-strand probes used to amplify RPP cDNA by PCR